# EUROPÄISCHE PATENTSCHRIFT

(11) **EP 3 966 476 B1**
(45) Veröffentlichungstag und Bekanntmachung des Hinweises auf die Patenterteilung: **11.06.2025**
(21) Anmeldenummer: 20720908.1
(22) Anmeldetag: 09.04.2020
(51) Int. Cl.: F16J 13/02, G21F 7/005, B65D 45/02, B01L 3/00, B01L 1/02, F16J 13/24

(54) **VORRICHTUNG ZUR STERILEN ÜBERGABE VON GUT ZWISCHEN EINEM BEHÄLTER UND EINEM ISOLATOR**
APPARATUS FOR STERILE TRANSFER OF MATERIAL BETWEEN A CONTAINER AND AN ISOLATOR
DISPOSITIF DE TRANSFERT STÉRILE DE PRODUIT ENTRE UN RÉCIPIENT ET UN ISOLATEUR

(30) Priorität: 10.05.2019 DE 102019003317
(43) Veröffentlichungstag der Anmeldung: 16.03.2022
(73) Patentinhaber: Atec Pharmatechnik Gmbh, 24966 Sörup (DE)
(72) Erfinder: MUMM, Hans-Werner, 24966 Sörup (DE)
(74) Vertreter: Weidner Stern Jeschke
(86) Internationale Anmeldenummer: PCT/EP2020/060234
(87) Internationale Veröffentlichungsnummer: WO 2020/229067

(56) Entgegenhaltungen:
- DE-T2- 69 307 433
- US-A- 5 425 400
- US-A1- 2003 126 799
- US-A1- 2016 201 382
- US-B1- 6 315 013
- US-B2- 9 704 607

## Beschreibung

Die Erfindung betrifft eine Vorrichtung zur sterilen Übergabe von Gut zwischen einem Behälter und einem Isolator gemäß dem Oberbegriff des Anspruchs 1.

Vorrichtungen dieser Art werden auch als Rapid Transfer Port bezeichnet. Rapid Transfer Ports umfassen einen im Wesentlichen aus dem Portflansch und der Porttür bestehenden Alpha-Teil in der Wand des Isolators und einen im Wesentlichen aus dem Behälterflansch und dem Behälterdeckel bestehenden Beta-Teil, der vor der Gutübergabe am Alpha-Teil angedockt wird. Ein solcher Rapid Transfer Port ist beispielsweise aus der US 9 704 607 B2 und aus der WO 2013/053844 A1 bekannt. Diese bekannte Vorrichtung besitzt insgesamt fünf Sicherheitseinrichtungen in Form von Bolzen, die verhindern, dass bei der Übergabe von Gut aus dem Behälter in den Isolator oder bei der Übergabe von Gut aus dem Isolator in den Behälter versehentlich eine Kontamination des Guts durch einen ungewollten Kontakt mit der Umgebung erfolgt oder umgekehrt toxische Substanzen aus dem Behälter und/oder Isolator in die Umgebung gelangen können, zum Beispiel infolge einer fehlerhaften Bedienung der Vorrichtung, die mit Hilfe der Sicherheitseinrichtungen verhindert werden soll.

Ähnliche Vorrichtungen dieser Art mit einer geringeren Anzahl von Sicherheitseinrichtungen sind aus der FR 2 695 343 A1 oder aus der WO 2015/032713 A1 bekannt.

Die erste Sicherheitseinrichtung verhindert ein Öffnen der Porttür, wenn der Behälter nicht mit einem Behälterdeckel versehen ist. Wenn die Porttür geschlossen ist und gegen den Behälterdeckel anliegt, befindet sich der Bolzen in seiner Freigabestellung, in der sich der Betätigungsmechanismus in die Offenstellung bringen lässt. Wenn die geschlossene Porttür nicht gegen einen Behälterdeckel anliegt, befindet sich der Bolzen in seiner Blockierstellung, in der er verhindert, dass der Betätigungsmechanismus in die Offenstellung gebracht werden kann.

Die zweite Sicherheitseinrichtung verhindert ein Öffnen der Porttür, solange der Behälterflansch nicht korrekt mit dem Portflansch verbunden, d.h. nicht ordnungsgemäß am Portflansch angedockt ist. In diesem Fall blockiert der Bolzen in seiner Blockierstellung den in der Schließstellung befindlichen Betätigungsmechanismus, so dass dieser die Schließstellung erst dann in Richtung der Offenstellung verlassen kann, wenn die Flansche des Behälters und des Isolators ordnungsgemäß miteinander verbunden sind. Zum Verbinden der beiden Flansche dient zumeist eine Bajonettverbindung, die korrekt hergestellt ist, wenn ein Nocken des Behälterflanschs gegen eine Schulter des Portflanschs anschlägt.

Die dritte Sicherheitseinrichtung verhindert, dass die beiden Flansche voneinander gelöst werden können, solange die Porttür geöffnet ist, d.h. während sich der Betätigungsmechanismus in seiner Offenstellung oder zwischen seiner Schließstellung und seiner Offenstellung befindet. In diesem Fall verhindert der Bolzen in seiner Blockierstellung ein Verdrehen der beiden Flansche, das zu einem Lösen der Bajonettverbindung führen könnte. Wenn sich der Betätigungsmechanismus in seiner Schließstellung befindet, befindet sich der Bolzen in seiner Freigabestellung, in der er das Verdrehen der beiden Flansche und das Lösen der Bajonettverbindung gestattet.

Die vierte Sicherheitseinrichtung dient dazu, ein Lösen des Behälterflanschs vom Portflansch zu verhindern, nachdem der Betätigungsmechanismus in die Offenstellung bewegt und die Porttür sowie der mit der Porttür verbundene Behälterdeckel geöffnet worden sind. In diesem Fall verhindert der Bolzen in der Blockierstellung die Rückkehr des Betätigungsmechanismus in seine Schließstellung, wenn er sich in seiner Offenstellung oder zwischen seiner Schließstellung und seiner Offenstellung befindet. Wenn sich der Betätigungsmechanismus in seiner Schließstellung befindet, befindet sich der Bolzen in seiner Freigabestellung.

Die in der US 9 704 607 B2 und WO 2013/053844 A1 ausführlich beschriebene fünfte Sicherheitseinrichtung wird nur sehr selten benötigt, nämlich nur im Fall einer gezielten Fehlbedienung oder einer völligen Unkenntnis des Bedieners von der Funktionsweise der Vorrichtung. Hier blockiert der Bolzen den Betätigungsmechanismus, wenn der Behälterflansch zwischen der Freigabe des Betätigungsmechanismus durch die zweite Sicherheitseinrichtung und der Blockierung des Behälterflanschs durch die dritte Sicherheitseinrichtung nicht ordnungsgemäß mit dem Portflansch verbunden ist.

Bei der Vorrichtung aus der US 9 704 607 B2 und WO 2013/053844 A1 ist der Bolzen der ersten Sicherheitseinrichtung axial beweglich in eine Durchgangsbohrung der Porttür eingesetzt, während der Bolzen der dritten Sicherheitseinrichtung und Teile der Bolzen der zweiten und fünften Sicherheitseinrichtung axial beweglich in eine Durchgangsbohrung des Portflanschs eingesetzt sind. Die Bolzen besitzen Durchmesser von wenigen Millimetern.

Wenn derartige Vorrichtungen auf pharmazeutischem oder auf medizinischem Gebiet eingesetzt werden, wo eine vollständige Sterilität des Guts im Inneren des Isolators und des Behälters gefordert wird, bildet der Alpha-Teil des Rapid Transfer Port eine Steril-Barriere, die weder von Partikeln oder Keimen durchdrungen werden darf. Da jedoch die Durchgangsbohrungen die Steril-Barriere durchdringen, müssen in die Durchgangsbohrung Dichtungen eingesetzt werden, die einen Hindurchtritt von Keimen durch die Durchgangsbohrungen verhindern sollen. Da sich die Bolzen jedoch in den Durchgangsbohrungen hin und her bewegen und in einer Endstellung mit ihren freien Enden aus den Bohrungen heraus ragen, können aber trotz der Dichtungen Partikel oder Keime durch die Durchgangsbohrungen hindurch gelangen. Zudem werden die Dichtungen durch die beweglichen Bolzen dynamisch beansprucht und können so schneller versagen. Auch lässt sich die Unversehrtheit der Dichtungen nur schwer kontrollieren, so dass im Falle eines Versagens einer Dichtung eine noch größere Gefahr einer Kontamination besteht. Weiter wird auch das Sterilisieren der Vorrichtung erschwert, da in die Durchgangsbohrungen eingedrungene Keime nicht oder nur unvollständig abgetötet werden können und so ebenfalls zu einer Kontamination führen können.

Weiterhin offenbaren die DE 693 07 433 T2, die US 2016/201382 sowie die US 5 425 400 A und die US 9704607B2 Portsysteme mit mechanischen Sicherheitseinrichtungen.

Ausgehend hiervon liegt der Erfindung die Aufgabe zugrunde, eine Vorrichtung der eingangs genannten Art dahingehend zu verbessern, dass im Bereich der Sicherheitseinrichtungen die Gefahr einer mögliche Kontamination des Isolators oder des Behälters reduziert und nach Möglichkeit verhindert werden kann.

Diese Aufgabe wird erfindungsgemäß dadurch gelöst, dass mindestens eine und vorzugsweise mehrere der Sicherheitseinrichtungen jeweils zwei Magnetelemente umfasst, die relativ zueinander beweglich sind, dass zwischen den beiden Magnetelementen mindestens eine geschlossene Wand ohne Durchgangsöffnung angeordnet ist und dass sich die Magnetelemente entweder in einer Blockierstellung oder in einer Freigabestellung der Sicherheitseinrichtung durch die geschlossene Wand hindurch gegenseitig anziehen oder abstoßen.

Da die mindestens eine geschlossene Wand ohne Durchgangsöffnung zwischen den beiden Magnetelementen für Keime undurchdringlich ist, kann anders als bei bekannten Sicherheitseinrichtungen eine Durchdringung der Steril-Barriere sicher verhindert werden. Die erfindungsgemäßen Sicherheitseinrichtungen lassen sich durch die mindestens eine geschlossene Wand zwischen den beiden Magnetelementen hindurch kontaktlos aktivieren, d.h. aus ihrer Blockierstellung in ihre Freigabestellung bringen, und umgekehrt. Dabei wird jeweils eines der beiden Magnetelemente so weit an das andere Magnetelement angenähert, dass die zwischen den beiden Magnetelementen wirkenden magnetischen Anziehungs- oder Abstoßungskräfte durch die Wand hindurch eine Bewegung des anderen Magnetelements hervorrufen, die bewirkt, dass die Sicherheitseinrichtung die Blockierstellung oder die Freigabestellung einnimmt.

Grundsätzlich ist es ausreichend, wenn zwischen den beiden Magnetelementen eine einzige geschlossene Wand angeordnet ist. Da die beiden Magnetelemente jedoch stets in unterschiedlichen und in Bezug zueinander beweglichen Bauteilen der Vorrichtung angeordnet sind, z.B. das eine im Portflansch und das andere im Betätigungsmechanismus oder das eine in der Porttür oder einem zusammen mit der Porttür beweglichen Bauteil und das andere im Portflansch, können zwischen den Magnetelementen bevorzugt zwei geschlossene Wände angeordnet sein, die jedes der beiden Bauteile zum Inneren des Isolators hin dicht abschließen.

Grundsätzlich ist es möglich, die Vorrichtung nur mit einer einzigen der fünf Sicherheitseinrichtungen auszustatten, wie z.B. mit der dritten Sicherheitseinrichtung, die ein Lösen des Behälters vom Isolator verhindert, solange die Porttür geöffnet ist. In diesem Fall wird diese Sicherheitseinrichtung mit zwei Magnetelementen ausgestattet.

Weiter ist es aber auch möglich, die Vorrichtung mit zwei, drei oder vier Sicherheitseinrichtungen auszustatten, wie z.B. die Vorrichtung aus der FR 2 695 343 A1. In diesem Fall können eine einzige oder auch mehrere der Sicherheitseinrichtungen mit zwei Magnetelementen ausgestattet werden. Vorzugsweise werden immer diejenigen Sicherheitseinrichtungen mit zwei Magnetelementen ausgestattet, bei denen ansonsten eine Durchgangsbohrung in einem der Bauteile erforderlich wäre.

Erfindungsgemäße Sicherheitseinrichtungen können unabhängig von der Konstruktion der Vorrichtung oder des Rapid Transfer Ports eingesetzt werden: Zum Beispiel gibt es Rapid Transfer Ports, wo der Betätigungsmechanismus bzw. ein drehbarer Schlossteil eines Schlosses desselben an der Innenseite des Portflanschs angebracht ist, wie zum Beispiel in der US 9 704 607 B2 beschrieben. Alternativ gibt es Rapid Transfer Ports, wo der Betätigungsmechanismus bzw. ein drehbarer Schlossteil eines Schlosses desselben zusammen mit der Porttür beweglich ist, wie nachfolgend beschrieben.

Erfindungsgemäße Sicherheitseinrichtungen können zudem nicht nur bei Rapid Transfer Ports eingesetzt werden, bei denen das Öffnen und das Schließen der Porttür sowie die Bedienung des Betätigungsmechanismus manuell erfolgen, z.B. mittels eines ins Innere des Isolators ragenden Handschuhs, sondern auch bei Rapid Transfer Ports, bei denen das Öffnen und das Schließen der Porttür sowie die Bedienung des Betätigungsmechanismus mit Hilfe motorischer Antriebe erfolgen, z.B. von der Außenseite des Isolators aus.

Eine vorteilhafte Ausgestaltung der Erfindung sieht vor, dass jedes der beiden Magnetelemente einer Sicherheitseinrichtung einen permanentmagnetischen Körper umfasst. Diese Ausgestaltung hat den Vorteil, dass wahlweise entweder mit Anziehungs- oder mit Abstoßungskräften zwischen den beiden Magnetelementen gearbeitet werden kann. Wenn nur mit Anziehungskräften zwischen den beiden Magnetelementen gearbeitet werden soll, ist es alternativ jedoch auch möglich, eines der beiden Magnetelemente mit einem ferromagnetischen Körper und das andere mit einem permanentmagnetischen Körper zu versehen. Grundsätzlich wäre es auch denkbar, dass mindestens eines der Magnetelemente als Elektromagnet ausgebildet ist, was jedoch eine Stromzufuhr erforderlich machen würde.

Für die Funktionsweise der Magnetelemente einer bestimmten Sicherheitseinrichtung gibt es grundsätzlich vier Alternativen:
1) Die Magnetelemente ziehen sich in der Blockierstellung gegenseitig an, nicht jedoch in der Freigabestellung,
2) Die Magnetelemente stoßen sich in der Blockierstellung gegenseitig ab, nicht jedoch in der Freigabestellung,
3) Die Magnetelemente ziehen sich in der Freigabestellung gegenseitig an, nicht jedoch in der Blockierstellung,
4) Die Magnetelemente stoßen sich in der Freigabestellung gegenseitig ab, nicht jedoch in der Blockierstellung.

Eine weitere vorteilhafte Ausgestaltung der Erfindung sieht vor, dass mindestens eines der beiden Magnetelemente innerhalb des Bauteils, in dem es montiert ist, z.B. dem Portflansch, der Porttür oder dem Betätigungsmechanismus, zwischen zwei Endstellungen beweglich ist, die der Blockierstellung bzw. Freigabestellung der jeweiligen Sicherheitseinrichtung entsprechen. In einigen Fällen reicht es aus, wenn nur eines der beiden Magnetelemente zwischen zwei Endstellungen beweglich ist, während es in anderen Fällen von Vorteil ist, wenn beide Magnetelemente zwischen zwei Endstellungen beweglich sind.

Da die beiden Magnetelemente erfindungsgemäß relativ zueinander beweglich sind, kann das eine Magnetelement an das andere Magnetelement angenähert oder von diesem weg bewegt werden. Diese Relativbewegung des einen Magnetelements in Bezug zum anderen Magnetelement kann durch eine Bewegung eines Bauteils der Vorrichtung ausgelöst werden, zum Beispiel durch eine Bewegung des Betätigungsmechanismus zwischen dessen Offen- und Schließstellung, durch eine Bewegung der Porttür beim Öffnen oder Schließen derselben oder durch eine Relativbewegung des Behälterflanschs und des Portflanschs beim Verbinden derselben.

Eine Annäherung des einen Magnetelements an das andere Magnetelement führt infolge der gegenseitigen Anziehungs- oder Abstoßungskräfte dazu, dass sich das andere Magnetelement in seine eine Endstellung bewegt, in der sich die zugehörige Sicherheitseinrichtung in der Freigabestellung oder der Blockierstellung befindet. Eine Wegbewegung vom anderen Magnetelement führt dazu, dass die Anziehungs- oder Abstoßungskräfte aufgehoben werden oder zumindest abnehmen.

Damit sich das andere Magnetelement auch in diesem Fall in seine andere Endstellung bewegt, so dass sich anschließend die zugehörige Sicherheitseinrichtung in der Blockierstellung bzw. der Freigabestellung befindet, ist das andere Magnetelement bevorzugt entgegen der Kraft einer Rückstellfeder beweglich, die das Magnetelement nach der Aufhebung oder Abnahme der gegenseitigen Anziehungs- oder Abstoßungskräfte in die andere Endstellung zurück bewegt.

Bei einer Reihe von bekannten Rapid Transfer Ports umfasst der Betätigungsmechanismus ein Schloss mit einem Schlossteil, der um eine Drehachse aus der Schließstellung in die Offenstellung drehbar ist und in der Regel im Inneren des Isolators entweder am Portflansch oder an einem radial über die Porttür überstehenden Bügel montiert und bei einer manuellen Bedienung des Betätigungsmechanismus einen zum Drehen des Schlossteils dienenden Betätigungsgriff aufweist, der von einem Bediener durch einen in den Isolator ragenden Handschuh ergriffen und betätigt werden kann. In diesem Fall ist es von Vorteil, wenn mindestens eines der beiden Magnetelemente parallel zur Drehachse des Schlossteils beweglich ist. Dies erleichtert es beim Vorhandensein von mehreren erfindungsgemäßen Sicherheitseinrichtungen deren Magnetelemente nebeneinander im drehbaren Schlossteil zu montieren und getrennt voneinander zu betätigen. Das bewegliche Magnetelement dient vorteilhaft dazu, in der Blockierstellung einer Sicherheitseinrichtung eine Drehung des Schlossteils zu blockieren und in der Freigabestellung eine Drehung des Schlossteils zuzulassen.

Vorteilhaft ist bei mindestens einer Sicherheitseinrichtung eines der beiden Magnetelemente im Portflansch angeordnet. Dabei kann es sich z.B. um die vierte Sicherheitseinrichtung handeln, die ein im Portflansch angeordnetes erstes Magnetelement und ein zusammen mit der Porttür bewegliches zweites Magnetelement umfasst. Das zweite Magnetelement gibt den Betätigungsmechanismus bzw. den drehbaren Schlossteil in der Freigabestellung der Sicherheitseinrichtung frei, wenn die Porttür gegen den Portflansch anliegt und sich die beiden Magnetelemente gegenseitig anziehen oder abstoßen. Umgekehrt blockiert das zweite Magnetelement den Betätigungsmechanismus bzw. den drehbaren Schlossteil, wenn die Porttür geöffnet wird und durch die Öffnungsbewegung die beiden Magnetelemente so weit auseinander bewegt werden, dass sie sich nicht mehr oder nicht mehr ausreichend stark gegenseitig anziehen oder abstoßen und demzufolge die Rückstellfeder das zweite Magnetelement in diejenige Endstellung bewegt, in der sich die sich die Sicherheitseinrichtung in der Blockierstellung befindet.

Es kann sich jedoch auch um die zweite Sicherheitseinrichtung handeln, die ebenfalls neben einem im Portflansch angeordneten ersten Magnetelement ein auf die Betätigungseinrichtung einwirkendes zweites Magnetelement umfasst. In diesem Fall gibt das letztere den Betätigungsmechanismus bzw. dessen drehbaren Schlossteil frei, wenn der Behälterflansch und der Portflansch korrekt und vollständig miteinander verbunden sind. In diesem Zustand wird eine im Portflansch angeordnete Klinke von einem Nocken des Behälterflanschs ganz niedergedrückt. Die Klinke wirkt wiederum auf das bewegliche erste Magnetelement ein und drückt diese in diejenige Endstellung, in der es das zweite Magnetelement durch die mindestens eine Wand hindurch anzieht oder abstößt.

Eine weitere vorteilhafte Ausgestaltung der Erfindung sieht vor, dass bei mindestens einer der Sicherheitseinrichtungen eines der beiden Magnetelemente zusammen mit dem drehbaren Schlossteil um dessen Drehachse drehbar ist. Vorteilhaft handelt es sich dabei um die dritte Sicherheitseinrichtung, die ein erstes Magnetelement im Portflansch und ein zweites Magnetelement im drehbaren Schlossteil umfasst, wobei sich das zweite Magnetelement beim Drehen des Schlossteils in die Offenstellung vom ersten Magnetelement weg bewegt. Das letztere bewegt sich daraufhin durch die Kraft einer Rückstellfeder in seine andere Endstellung, in der es ein Zurückdrehen des mit dem Portflansch verbundenen Behälterflanschs blockiert.

An Stelle eines Schlosses mit einem drehbaren Schlossteil könnte der Betätigungsmechanismus auch ein Schloss mit einem linear beweglichen Schlossteil umfassen. Vorteilhaft wäre dann das zweite Magnetelement der dritten Sicherheitseinrichtung zusammen mit dem linear beweglichen Schlossteil beweglich, um es an das erste Magnetelement im Portflansch anzunähern, während die zweiten Magnetelemente der ersten und der zweiten Sicherheitseinrichtung den linear beweglichen Schlossteil blockieren bzw. freigeben würden.

Im Folgenden wird die Erfindung anhand eines in der Zeichnung schematisch dargestellten Ausführungsbeispiels näher erläutert.
Fig. 1 zeigt eine perspektivische Ansicht eines Portflanschs und einer Porttür einer erfindungsgemäßen Vorrichtung vor dem Andocken eines Behälters, von dem nur der Behälterflansch und der Behälterdeckel dargestellt sind;
Fig. 2 zeigt eine perspektivische Ansicht entsprechend Fig. 1 jedoch nach dem Andocken des Behälters;
Fig. 3 zeigt eine Draufsicht auf die entgegengesetzte Seite des Portflanschs und der Porttür vor dem Öffnen eines Schlosses des Betätigungsmechanismus;
Fig. 4 zeigt eine Ansicht entsprechend Fig. 3, jedoch nach dem Öffnen des Schlosses;
Fig. 5 zeigt eine Schnittansicht entlang der Linie A-A der Fig. 3 vor dem Andocken des Behälters und im Schließzustand des Schlosses;
Fig. 6 zeigt eine vergrößerte Ansicht des Details B der Fig. 5;
Fig. 7 zeigt eine Schnittansicht entlang der Linie A-A der Fig. 3 nach dem Andocken des Behälters und im Schließzustand des Schlosses;
Fig. 8 zeigt eine vergrößerte Ansicht des Details C der Fig. 7;
Fig. 9 zeigt eine weitere Schnittansicht entlang der Linie A-A der Fig. 3 nach dem Andocken des Behälters und im Schließzustand des Schlosses;
Fig. 10 zeigt eine vergrößerte Ansicht des Details D der Fig. 9;
Fig. 11 zeigt eine Schnittansicht entlang der Linie E-E der Fig. 4 nach dem Andocken des Behälters, im Öffnungszustand des Schlosses und vor dem Öffnen der Porttür;
Fig. 12 zeigt eine vergrößerte Ansicht des Details F der Fig. 11;
Fig. 13 zeigt eine vergrößerte Ansicht des Details G der Fig. 1 in einem Zustand vor dem Andocken des Behälters;
Fig. 14 zeigt eine vergrößerte Ansicht des Details G der Fig. 1 in einem Zustand nach dem Andocken des Behälters (in Fig. 10 nicht dargestellt);
Fig. 15 zeigt eine Schnittansicht entlang der Linie E-E der Fig. 4 nach dem Andocken des Behälters, im Öffnungszustand des Schlosses und vor dem Öffnen der Porttür;
Fig. 16 zeigt eine vergrößerte Ansicht des Details H der Fig. 15;
Fig. 17 zeigt eine perspektivische Schnittansicht entlang der Linie E-E der Fig. 4 nach dem Andocken des Behälters, im Öffnungszustand des Schlosses und bei spaltbreit geöffneter Porttür;
Fig. 18 zeigt eine vergrößerte Ansicht des Details I der Fig. 17;
Fig. 19 zeigt eine teilweise geschnittene Seitenansicht des Portflanschs und der Porttür nach dem Öffnen des Schlosses und bei spaltbreit geöffneter Porttür;
Fig. 20 zeigt eine vergrößerte Ansicht des Details K der Fig. 15.

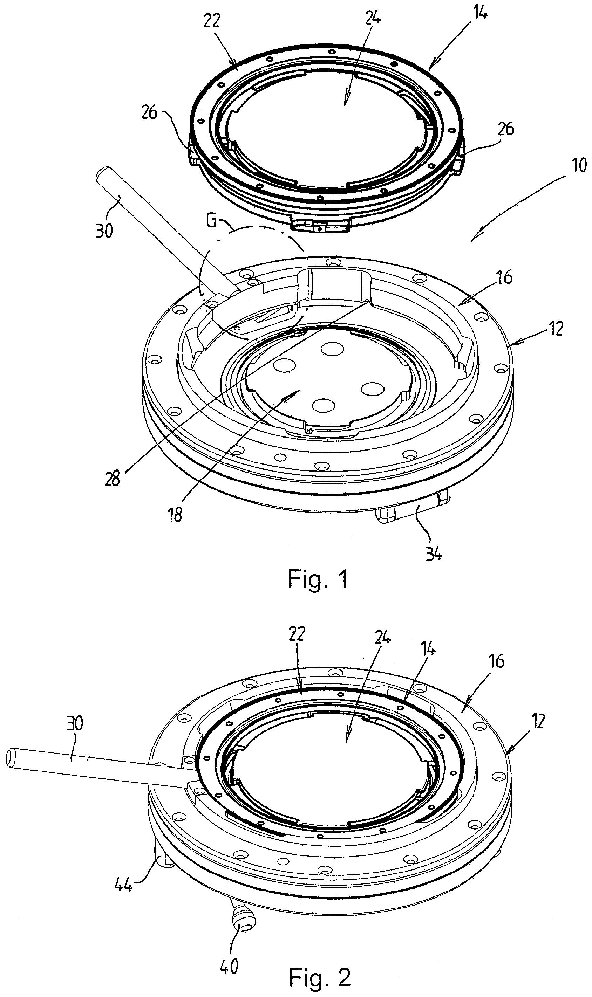

Die in der Zeichnung nur teilweise dargestellte und auf dem Fachgebiet auch als Rapid Transfer Port bezeichnete Vorrichtung 10 dient zur sterilen Übergabe von Gut zwischen einem Behälter (nicht dargestellt) und einem Isolator (nicht dargestellt). Bei dem Gut kann es sich zum Beispiel um Verschlussstopfen aus Gummi oder Kunststoff für Infusionsflaschen oder Vials, um Teile von Spritzen oder auch um Flüssigkeiten handeln. Zur Erläuterung des Übergabevorgangs wird auf die Fig. 1 der US 9 704 607 B2 und die zugehörige Beschreibung verwiesen.

Zur sterilen Gutübergabe umfasst die Vorrichtung 10 auf der Seite des Isolators einen sogenannten Alpha-Teil 12 oder Alpha-Port, während sie auf der Seite des Behälters einen sogenannten Beta-Teil 14 oder Beta-Port umfasst. Der Alpha-Teil 12 besitzt eine Portöffnung, die von einem auch als Alpha-Flansch bezeichneten Portflansch 16 umgeben ist, eine Porttür 18 zum Verschließen der Portöffnung sowie einen Betätigungsmechanismus 20 zum Öffnen und Schließen der Porttür 18. Der Beta-Teil besitzt eine Behälteröffnung, die von einem auch als Beta-Flansch bezeichneten Behälterflansch 22 umgeben ist, sowie einen Behälterdeckel 24, der normalerweise die Behälteröffnung verschließt und vom Behälterflansch 22 abgenommen werden kann. Zur Übergabe des Gutes wird der Beta-Teil 14 des Behälters am Alpha-Teil 12 des Isolators angedockt. Dies erfolgt mit zwei Bajonettverbindungen die den Behälterflansch 22 mit dem Portflansch 16 und den Behälterdeckel 24 dicht mit der Porttür 18 verbinden, während sich gleichzeitig der Behälterdeckel 24 vom Behälterflansch 22 löst. Die eine Bajonettverbindung weist radial über den Behälterflansch 22 überstehende Nocken 26 auf, die beim Andocken mit einer entsprechenden Aufnahmenut 28 des Portflanschs 16 in Eingriff treten. Zum vollständigen Andocken des Beta-Teils 14 am Alpha-Teil 12 wird der innere Teil des Portflanschs 16 mittels eines Hebels 30 bis zum Anschlagen der Nocken 26 gegen entsprechende Nockenanschläge in der Nut 28 um eine zur Ebene der Portöffnung senkrechte Achse gedreht.

Bei anderen Rapid Transfer Ports kann das Andocken des Beta-Teils 14 am Alpha-Teil 12 auch durch eine Drehung des Beta-Teils 14 in Bezug zum Alpha-Teil 12 erfolgen.

Die Porttür 18 ist schwenkbar am Portflansch 16 montiert, so dass sie sich nach dem Andocken des Behälters zusammen mit dessen Deckel 24 ins Innere des Isolators schwenken lässt. Dazu ist die Porttür 18 mit einem starr an der Porttür 18 montierten Bügel 32 versehen, der sich diametral über die Innenseite der Porttür 18 erstreckt. Ein erstes radial über den Umfang der Porttür 18 überstehendes Ende des Bügels 32 ist mittels eines Schwenkgelenks 34 schwenkbar an der Innenseite des Portflanschs 16 angelenkt.

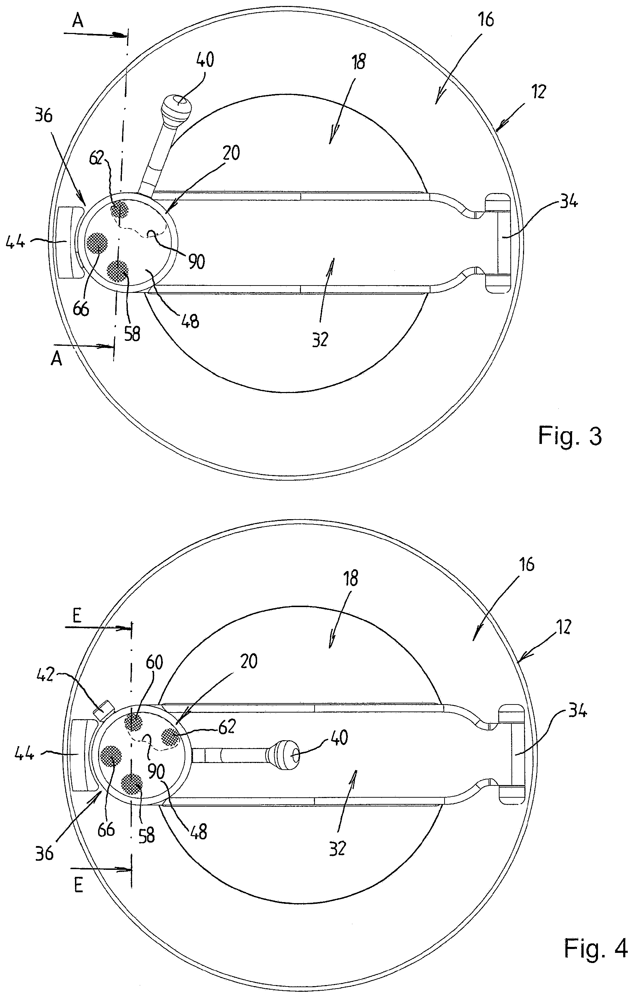

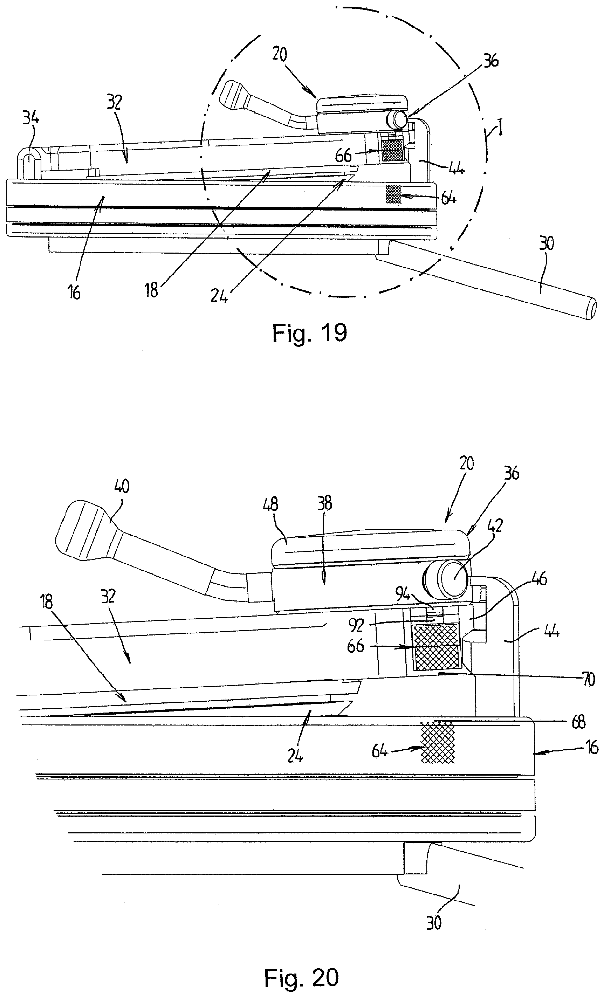

Die Porttür 18 kann in ihrer geschlossenen Stellung mittels des Betätigungsmechanismus 20 verriegelt werden, der zu diesem Zweck ein Schloss 36 umfasst. Das Schloss 36 umfasst einen ersten Schlossteil 38, der drehbar auf einem zweiten überstehenden Ende des Bügels 32 montiert ist, mittels eines Betätigungsgriffs 40 zwischen einer Schließstellung (Fig. 3) und einer Offenstellung (Fig. 4) gedreht werden kann und eine radial überstehende Verriegelungsnase 42 aufweist. Das Schloss 36 umfasst weiter einen zweiten Schlossteil 44, der gegenüber vom ersten Schlossteil 38 starr an der Innenseite des Portflanschs 16 montiert ist und eine Riegelaufnahme 46 (Fig. 20) aufweist, in welche die Verriegelungsnase 42 in der Schließstellung eingreift, um die Porttür 18 geschlossen zu halten. In der Offenstellung ist die Verriegelungsnase 42 aus der Riegelaufnahme 46 ausgerückt.

Der drehbare Schlossteil 38 besitzt eine scheibenförmige Gestalt und wird von einer senkrecht über den Bügel 32 überstehenden starren Achse (nicht sichtbar) durchsetzt. Auf den Schlossteil 38 ist eine scheibenförmige Abdeckung 48 aufgerastet, die sich zusammen mit dem Schlossteil 38 dreht.

Die Vorrichtung 10 umfasst vier im Alpha-Teil 12 angeordnete Sicherheitseinrichtungen 50, 52, 54, die eine Fehlbedienung des Rapid Transfer Port verhindern sollen, durch die bei der Übergabe von Gut eine Kontamination des Guts durch einen ungewollten Kontakt mit der Umgebung oder eine Kontamination des Isolators durch einen ungewollten Eintritt von Keimen verursacht werden könnte.

Dabei verhindert die erste Sicherheitseinrichtung ein Öffnen der Porttür 18, wenn der Behälter nicht mit einem Behälterdeckel 24 versehen und daher das Innere des Behälters nicht steril ist. Die zweite Sicherheitseinrichtung 50 verhindert eine Bewegung des drehbaren Schlossteils 38 in die Offenstellung und damit ein Öffnen der Porttür 18, solange der Behälterflansch 14 nicht ordnungsgemäß am Portflansch 12 angedockt ist. Die dritte Sicherheitseinrichtung 52 blockiert den ordnungsgemäß mit dem Portflansch 12 verbundenen Behälterflansch 14 und verhindert ein Lösen der zugehörigen Bajonettverbindung, sobald der drehbare Schlossteil 38 die Schließstellung in Richtung der Offenstellung verlässt. Die vierte Sicherheitseinrichtung 54 verhindert bei geöffneter Porttür 18 ein Zurückdrehen des Schlossteils 38 in die Schließstellung und damit auch die Freigabe der zweiten und der dritten Sicherheitseinrichtung 50, 52.

Um zu vermeiden, dass die Sicherheitseinrichtungen 50, 52, 54 die Sterilität des Rapid Transfer Port beeinträchtigen und die Steril-Barriere des Alpha-Teils 12 von Keimen durchdrungen werden kann. umfassen die drei Sicherheitseinrichtungen 50, 52, 54 jeweils zwei Magnetelemente 56, 58; 60, 62; 64, 66, die relativ zueinander beweglich sind, wobei zwischen den beiden Magnetelementen 56, 58; 60, 62; 64, 66 zwei geschlossene Wände 68, 70 ohne Durchgangsöffnung angeordnet sind und wobei sich die Magnetelemente 56, 58; 60, 62; 64, 66 entweder in einer Blockierstellung oder in einer Freigabestellung der jeweiligen Sicherheitseinrichtung 50, 52, 54 durch die geschlossenen Wände 68, 70 hindurch gegenseitig anziehen oder abstoßen.

Bei allen drei Sicherheitseinrichtungen 50, 52, 54 ist eines 56; 60; 64 der beiden Magnetelemente 56, 58; 60, 62; 64, 66, nachfolgend erstes Magnetelement genannt, im Portflansch 16 untergebracht, während das andere 58; 62; 66 der beiden Magnetelemente, nachfolgend zweites Magnetelement genannt, im Bügel 32 der Porttür 18 untergebracht ist. Beide Magnetelemente 56, 58; 60, 62; 64, 66 umfassen jeweils einen zylindrischen oder ringförmigen Magnetkörper aus einem permanentmagnetischen Material, zum Beispiel einer Neodym-Eisen-Bor-Legierung oder einer anderen Seltenerdlegierung. Die beiden Magnetkörper sind jeweils so montiert, dass sich zwei ebene Stirnseiten der Magnetkörper gegenüberliegen.

Die zur Abfrage des Vorhandenseins des Behälterdeckels 24 dienende erste Sicherheitseinrichtung kommt bei dem hier beschriebenen Rapid Transfer Port anders als bei dem Rapid Transfer Port aus der US 9 704 607 B2 nur mit einer statisch beanspruchten Dichtung in der Porttür 18 aus. Deshalb, und um zu vermeiden, dass jeder Behälterdeckel mit einem Magnetelement bestückt werden muss, wird sie hier ohne Magnetelemente ausgeführt und soll nicht näher beschrieben werden. Die oben genannte zweite, dritte und vierte Sicherheitseinrichtung 50, 52, 54 umfassen hingegen jeweils zwei Magnetelemente 56, 58; 60, 62; 64, 66.

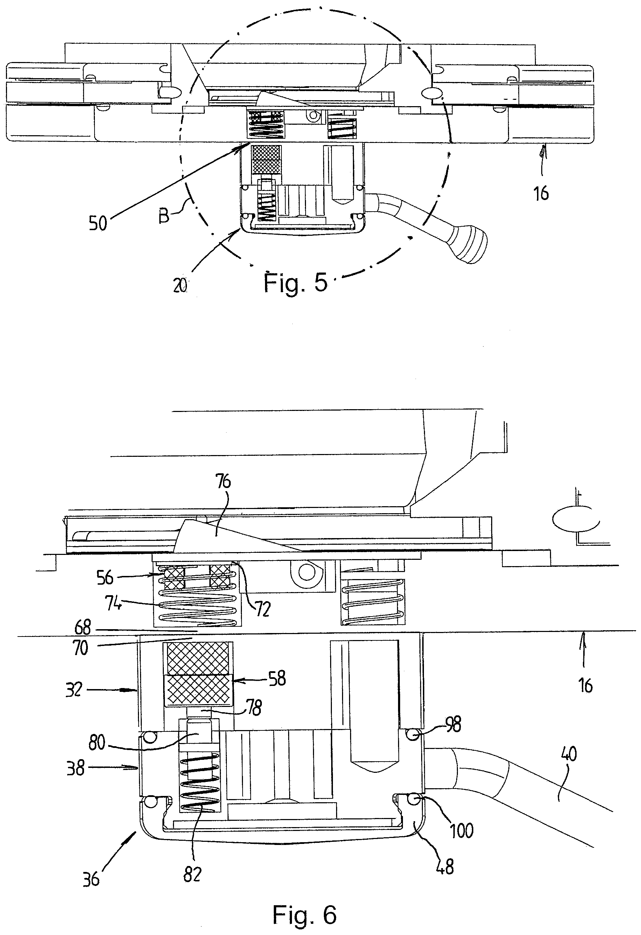

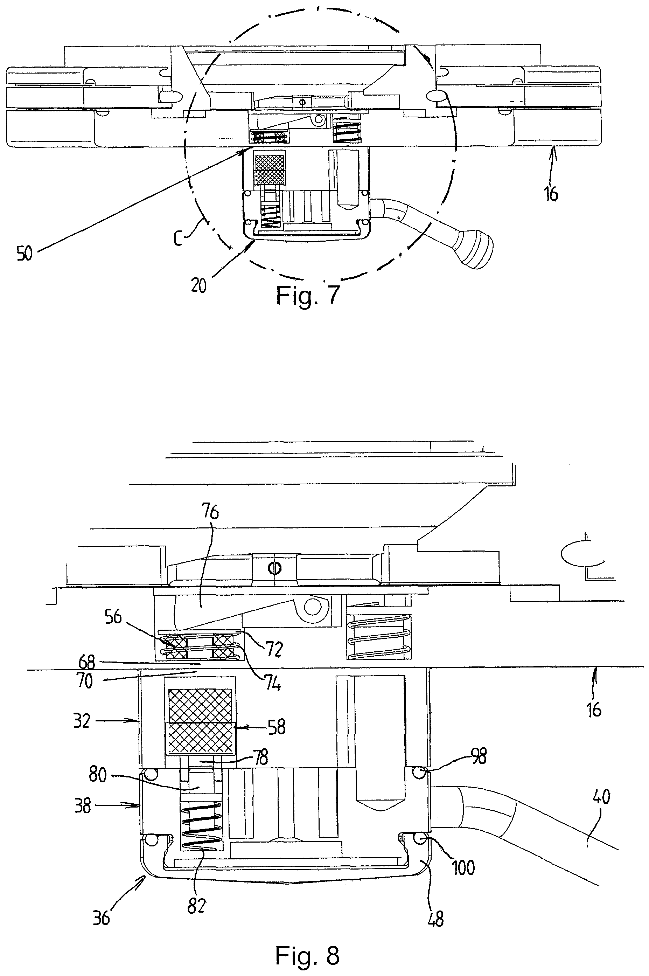

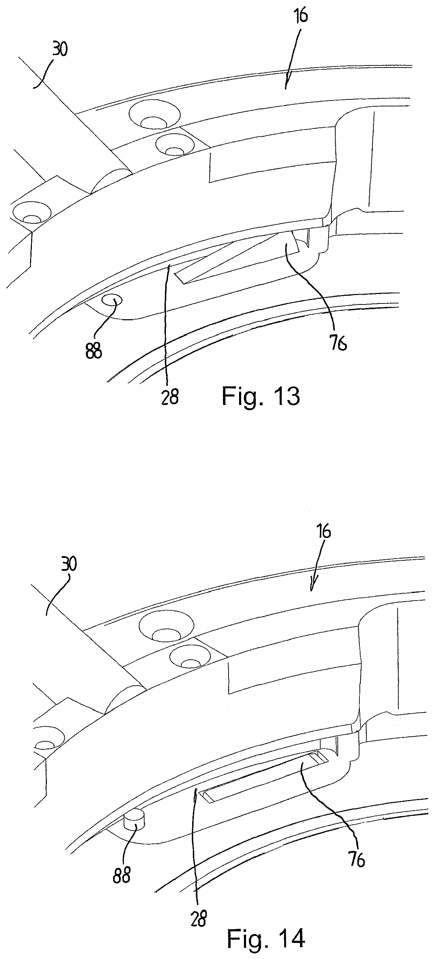

Wie am besten in den Figuren 5 bis 8, 13 und 14 dargestellt, besteht bei der zweiten Sicherheitseinrichtung 50 das im Portflansch 16 montierte erste Magnetelement 56 aus einem ringförmigen Magnetkörper und einer Halterung 72, die in einer Sacklochbohrung des Portflanschs 16 entgegen der Kraft einer Rückstellfeder 74 beweglich sind. Die Halterung 72 umfasst eine Platte und einen Stift, der den ringförmigen Magnetkörper durchsetzt. Auf die Platte wirkt eine nahe der Sacklochbohrung schwenkbar im Portflansch 16 gelagerte Klinke 76 ein. Die Klinke 76 ragt vor dem Andocken des Behälters in die Aufnahmenut 28 des Portflanschs 16, wie in Fig. 13 dargestellt, wo sie von einem der Nocken 26 des Behälterflanschs 22 vollständig niedergedrückt wird, wie in Fig. 14 dargestellt, wenn die beiden Flansche 22, 16 beim Anschlagen der Nocken 26 gegen einen Anschlag des Portflanschs 16 ordnungsgemäß miteinander verbunden sind. Nach dem Abdocken des Behälters wird die Klinke 76 von der Rückstellfeder 74 wieder in die Aufnahmenut 28 bewegt, wie in Fig. 13 dargestellt. In Abhängigkeit von der Schwenkstellung der Klinke 76 wird das erste Magnetelement 56 zwischen zwei Endstellungen hin und her bewegt, wie in Fig. 6 und 8 dargestellt. Wenn die Klinke 76 in die Aufnahmenut 28 ragt, befindet sich das erste Magnetelement 56 in der ersten Endstellung (Fig. 6), während es sich bei niedergedrückter Klinke 76 in der zweiten Endstellung am Grund der Sacklochbohrung befindet (Fig. 8). Die Sacklochbohrung ist zum Bügel 32 hin durch die Wand 68 verschlossen, die aus dünnem nicht-rostendem und nicht-magnetischem Stahlblech besteht.

Das zweite Magnetelement 58 befindet sich gegenüber vom ersten Magnetelement 56 im Inneren des Bügels 32. Neben einem zylindrischen Magnetkörper umfasst das zweite Magnetelement 58 einen Stift 78, der über die vom Portflansch 16 abgewandte Seite des Magnetkörpers übersteht. Der Magnetkörper und der Stift 78 sind in einer Sacklochbohrung des Bügels 32 beweglich, die zum Portflansch hin durch die Wand 70 verschlossen ist. Die Wand 70 besteht ebenfalls aus dünnem nicht-rostendem und nicht-magnetischem Stahlblech.

Wie am besten in Fig. 6 und 8 dargestellt, liegt der Stift 78 mit seinem vom Magnetkörper abgewandten Stirnende gegen einen weiteren Stift 80 an, der entgegen der Kraft einer Rückstellfeder 82 in einer Bohrung des drehbaren Schlossteils 38 axial verschiebbar gelagert ist. Die Rückstellfeder 82 drückt das zweite Magnetelement 58 über die Stifte 80, 78 gegen die Wand 70, wenn sich das erste Magnetelement 56 bei unbelasteter Klinke 76 in seiner ersten Endstellung befindet.

Die beiden Magnetkörper sind so ausgerichtet, dass sie sich gegenseitig abstoßen. Wenn die Klinke 76 niedergedrückt und dadurch das erste Magnetelement 56 in die zweite Endstellung bewegt und an das zweite Magnetelement 58 angenähert wird, wird das zweite Magnetelement 58 entgegen der Kraft der Feder 82 vom Grund der Sacklochbohrung abgehoben. Die Abstoßungskräfte der beiden Magnetelemente 56, 58 sind so auf die Federkraft der Rückstellfeder 82 abgestimmt, dass die gegeneinander anliegenden ebenen Stirnflächen der beiden Stifte 78, 80 mit einer ebenen Anlagefläche des drehbaren Schlossteils 38 am Bügel 32 fluchten, wenn die Klinke 76 ganz niedergedrückt ist. Diese in Fig. 8 dargestellte Stellung ist die Freigabestellung der zweiten Sicherheitseinrichtung 50, in der sich der drehbare Schlossteil 38 aus der Schließstellung des Schlosses 36 in dessen Offenstellung drehen lässt.

Wenn sich die Klinke 76 beim Abdocken des Behälters wieder in die in Fig. 6 und 13 dargestellte Stellung zurückbewegt, nimmt die zweite Sicherheitseinrichtung 50 die Blockierstellung ein, in der sich der drehbare Schlossteil 38 nicht aus der Schließstellung herausbewegen lässt.

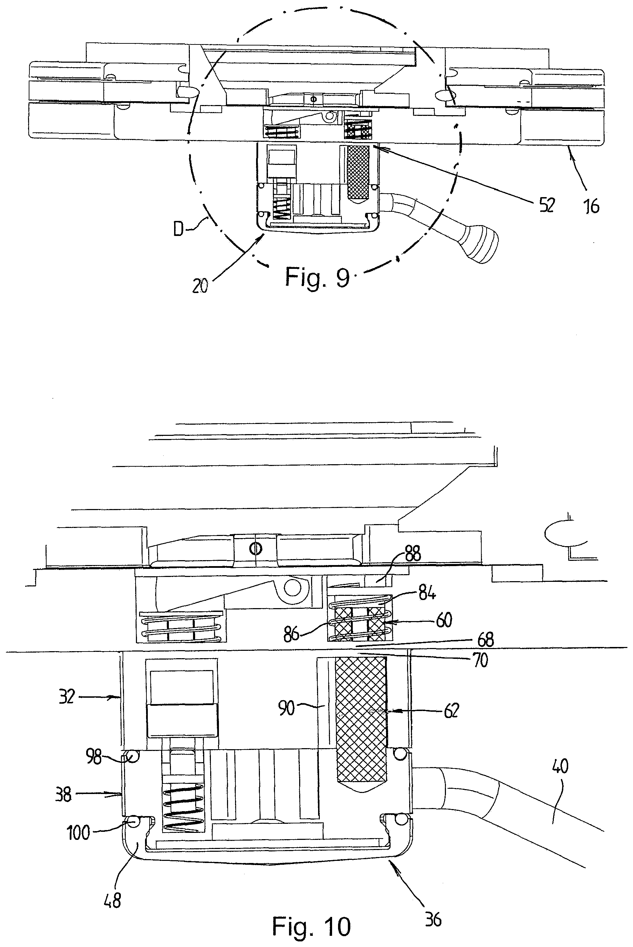

Wie am besten in den Figuren 9 bis 12 dargestellt, weist bei der dritten Sicherheitseinrichtung 52 das im Portflansch 16 montierte erste Magnetelement 60 eine entsprechende Ausbildung wie bei der zweiten Sicherheitseinrichtung 50 auf und besteht aus einem ringförmigen Magnetkörper und einer Halterung 84, die in einer Sacklochbohrung des Portflanschs 16 entgegen der Kraft einer Rückstellfeder 86 beweglich sind. Die Halterung 84 besteht aus einer Platte und zwei Stiften, von denen der eine den ringförmigen Magnetkörper durchsetzt. Der andere Stift steht über die entgegengesetzte Seite der Platte über. Der Magnetkörper und die beiden Stifte sind in der Sacklochbohrung axial beweglich, die zum Bügel 32 hin durch die Wand 68 verschlossen ist.

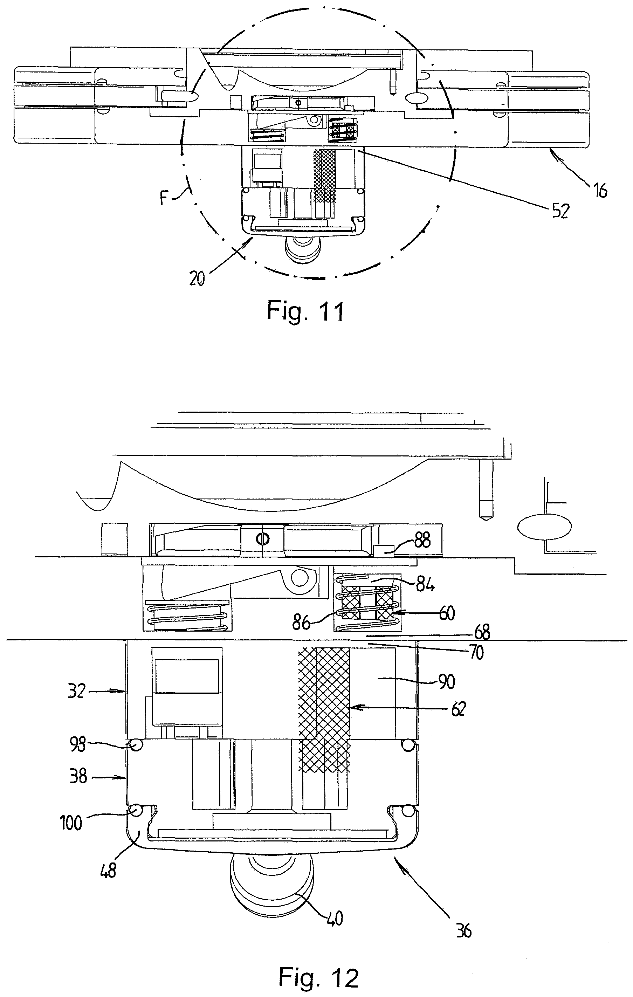

Das zweite Magnetelement 62 besteht aus einem langen zylindrischen Magnetkörper, der drehfest mit dem drehbaren Schlossteil 38 verbunden ist, über die dem Portflansch 16 zugewandte Seite des Schlossteils 38 übersteht und in ein gebogenes Langloch 90 des Bügels 32 ragt, das an seinem Grund zum Portflansch 16 hin durch die Wand 70 verschlossen ist. Wenn sich der drehbare Schlossteil 38 in seiner Schließstellung befindet, wie in Fig. 3 und 10 dargestellt, ist das zweite Magnetelement 62 genau gegenüber von dem ersten Magnetelement 60 angeordnet. Wenn sich der drehbare Schlossteil 38 in seiner Offenstellung befindet, wie in Fig. 4 und 12 dargestellt, ist das zweite Magnetelement 62 in Bezug zum ersten Magnetelement 60 seitlich versetzt.

Die beiden Magnetkörper sind so ausgerichtet, dass sie sich gegenseitig anziehen, wenn sich in der Schließstellung des Schlosses 36 das zweite Magnetelement 62 gegenüber von dem ersten Magnetelement 60 befindet. Die Anziehungskräfte der beiden Magnetelemente 60, 62 sind so auf die Federkraft der Rückstellfeder 86 abgestimmt, dass in dieser Freigabestellung der dritten Sicherheitseinrichtung 52 das erste Magnetelement 60 eine erste Endstellung einnimmt, in welcher der Stift 88 entgegen der Kraft der Rückstellfeder 86 vollständig aus der Aufnahmenut 28 zurückgezogen ist (Fig. 13). Wenn das zweite Magnetelement 62 beim Drehen des Schlossteils 38 in die Offenstellung in Drehrichtung innerhalb des Langlochs 90 vom ersten Magnetelement 60 weg bewegt wird, dann nehmen die magnetischen Anziehungskräfte zwischen den Magnetelementen 60, 62 so weit ab, dass das erste Magnetelement 60 von der Rückstellfeder 86 wieder in die zweite Endstellung gedrückt wird, in welcher der Stift 88 wieder in die Aufnahmenut 28 ragt (Fig. 14) und sich die dritte Sicherheitseinrichtung 52 in ihrer Blockierstellung befindet. Die Position des Stiftes 88 in der Nut 28 ist so gewählt, dass er unmittelbar hinter einem der Nocken 26 des Behälterflanschs 22 in die Nut 28 ragt und ein Zurückdrehen des Nockens 26 bzw. des Behälterflanschs 22 verhindert.

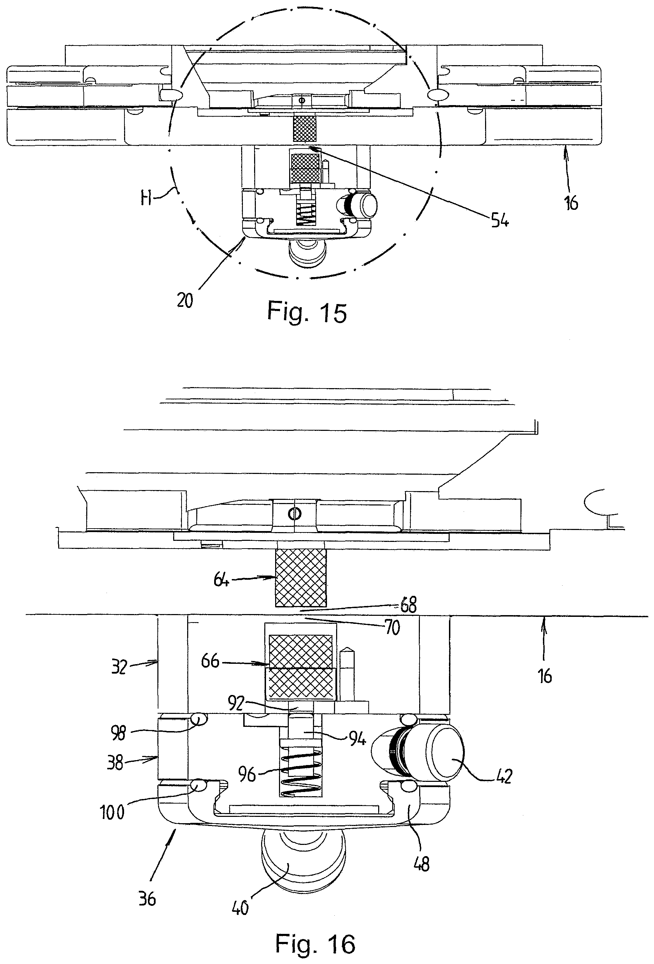

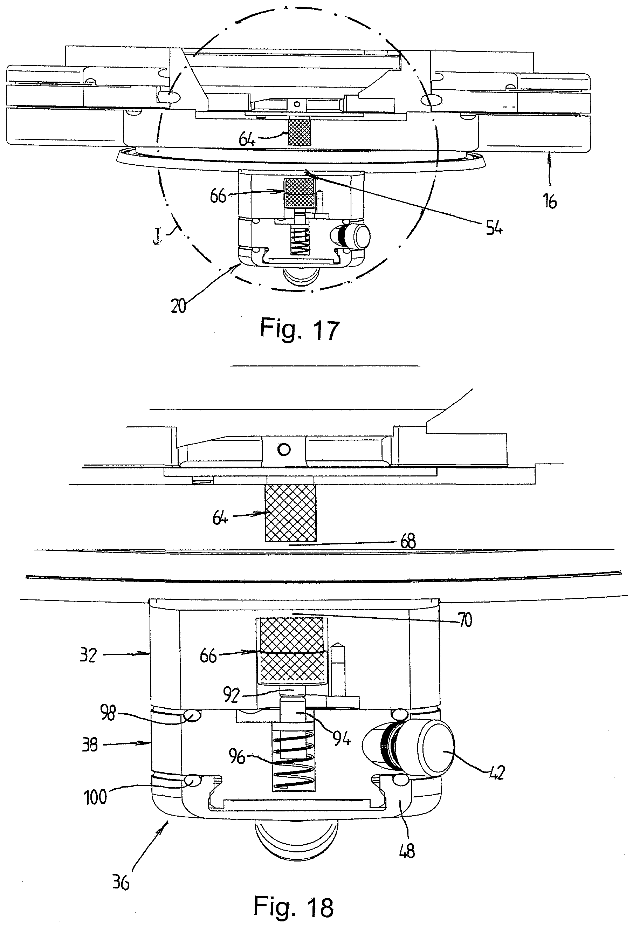

Wie am besten in den Figuren 15 bis 20 dargestellt, besteht bei der vierten Sicherheitseinrichtung 54 das erste Magnetelement 64 lediglich aus einem im Portflansch 16 montierten ortsfesten Magnetkörper, der an der Innenseite des Portflanschs 16 durch die Wand 68 vom Inneren des Isolators getrennt ist. Das zweite Magnetelement 66 befindet sich direkt gegenüber vom ersten Magnetelement 64 im Inneren des Bügels 32, so dass es sich beim Öffnen der Porttür 18 mit dieser vom ersten Magnetelement 64 wegbewegt und sich beim Schließen der Porttür 18 mit dieser an das erste Magnetelement 64 annähert. Das zweite Magnetelement 66 umfasst neben dem Magnetkörper einen Stift 92, der über die vom Portflansch 16 abgewandte Seite des Magnetkörpers übersteht. Der Magnetkörper und der Stift 92 sind in einer Sacklochbohrung des Bügels 32 axial beweglich, die zum Portflansch 16 hin durch die Wand 70 verschlossen ist. Wie am besten in Fig. 16 und 18 dargestellt, liegt der Stift 92 mit seinem vom Magnetkörper abgewandten ebenen Stirnende gegen das ebene Stirnende eines weiteren Stifts 94 an, der entgegen der Kraft einer Rückstellfeder 96 in einer Sacklochbohrung des drehbaren Schlossteils 38 axial verschiebbar gelagert ist.

Die beiden Magnetkörper sind so ausgerichtet, dass sie sich gegenseitig abstoßen, wenn die Porttür 18 geschlossen wird und damit das zweite Magnetelement 66 an das erste Magnetelement 64 angenähert wird. Die Abstoßungskräfte der beiden Magnetelemente 64, 66 sind derart auf die Federkraft der Rückstellfeder 96 abgestimmt, dass die gegeneinander anliegenden ebenen Stirnflächen der beiden Stifte 92, 94 mit der ebenen Anlagefläche des drehbaren Schlossteils 38 am Bügel 32 fluchten, wenn der Bügel 32 bei geschlossener Porttür 18 gegen den Portflansch 16 anliegt. Diese Stellung ist die Freigabestellung der vierten Sicherheitseinrichtung 54, in der sich der drehbare Schlossteil 38 aus der Offenstellung des Schlosses 36 zurück in die Schließstellung drehen lässt.

Wenn die Porttür 18 in der Offenstellung des Schlosses 36 spaltbreit geöffnet wird, nehmen die Abstoßungskräfte zwischen den beiden Magnetelementen 64, 66 ab. Dies hat zur Folge, dass die Kraft der Rückstellfeder 96 das zweite Magnetelement 66 innerhalb der Bohrung in Richtung der Wand 70 bewegt, wodurch sich die gegeneinander anliegenden ebenen Stirnflächen der beiden Stifte 92, 94 aus der Ebene der Anlagefläche des drehbaren Schlossteils 38 am Bügel 32 heraus bewegen, wie am besten in Fig. 18 dargestellt. In dieser Blockierstellung der vierten Sicherheitseinrichtung 54 lässt sich der drehbare Schlossteil 38 nicht aus der Offenstellung des Schlosses 36 in dessen Schließstellung drehen.

Wie am besten in den Figuren 6, 8, 10, 12, 16 und 18 dargestellt, ist der drehbare Schlossteil 38 zum Bügel 32 und zur Abdeckung 48 hin jeweils durch einen O-Ring 98, 100 abgedichtet, der sich entlang des äußeren Umfangs des drehbaren Schlossteils 38 bzw. der Abdeckung 48 erstreckt.

## Patentansprüche

1. Vorrichtung (10) zur sterilen Übergabe von Gut zwischen einem Behälter und einem Isolator, wobei der Behälter eine von einem Behälterflansch (22) umgebende Behälteröffnung und einen vom Behälterflansch (22) abnehmbaren Behälterdeckel (24) zum Verschließen der Behälteröffnung aufweist, wobei der Isolator eine von einem Portflansch (16) umgebende Portöffnung, eine Porttür (18) zum Verschließen der Portöffnung und einen zwischen einer Schließstellung und einer Offenstellung beweglichen Betätigungsmechanismus (20) zum Öffnen und Schließen der Porttür (18) aufweist, wobei der Behälterflansch (22) lösbar mit dem Portflansch (16) und der Behälterdeckel (24) lösbar mit der Porttür (18) verbindbar ist, sowie mit einer oder mehrerer der nachfolgenden Sicherheitseinrichtungen (50, 52, 54):
- einer ersten Sicherheitseinrichtung, die den Betätigungsmechanismus (20) in der Schließstellung blockiert und ihn freigibt, wenn die Porttür (18) gegen den Behälterdeckel (24) anliegt,
- einer zweiten Sicherheitseinrichtung (50), die den Betätigungsmechanismus (20) in der Schließstellung blockiert und ihn freigibt, wenn der Behälterflansch (22) korrekt mit dem Portflansch (16) verbunden ist,
- einer dritten Sicherheitseinrichtung (52), die den mit dem Portflansch (16) verbundenen Behälterflansch (22) blockiert, wenn der Betätigungsmechanismus (20) die Schließstellung verlässt, und die das Lösen der beiden Flansche (16, 22) voneinander verhindert, solange die Porttür (18) geöffnet ist,
- einer vierten Sicherheitseinrichtung (54), die eine Rückkehr des Betätigungsmechanismus (20) aus der Offenstellung in die Schließstellung blockiert, wenn die Porttür (18) geöffnet ist, und
- einer fünften Sicherheitseinrichtung, die den Betätigungsmechanismus (20) blockiert, wenn der Behälterflansch (22) zwischen der Freigabe des Betätigungsmechanismus (20) durch die zweite Sicherheitseinrichtung (50) und der Blockierung des Behälterflanschs (22) durch die dritte Sicherheitseinrichtung (52) nicht korrekt mit dem Portflansch (16) verbunden ist, **dadurch gekennzeichnet, dass** mindestens eine der Sicherheitseinrichtungen (50; 52; 54) zwei Magnetelemente (56, 58; 60, 62; 64, 66) umfasst, die relativ zueinander beweglich sind, dass zwischen den beiden Magnetelementen (56, 58; 60, 62; 64, 66) mindestens eine geschlossene Wand (68, 70) ohne Durchgangsöffnung angeordnet ist und dass sich die Magnetelemente (56, 58; 60, 62; 64, 66) entweder in einer Blockierstellung oder in einer Freigabestellung der Sicherheitseinrichtung (50; 52; 54) durch die geschlossene Wand (68, 70) hindurch gegenseitig anziehen oder abstoßen.

2. Vorrichtung nach Anspruch 1, **dadurch gekennzeichnet, dass** die beiden Magnetelemente (56, 58; 60, 62; 64, 66) in zwei durch die mindestens eine Wand (68, 70) getrennten und in Bezug zueinander beweglichen Bauteilen (32; 16) der Vorrichtung angeordnet sind.

3. Vorrichtung nach Anspruch 1 oder 2, **dadurch gekennzeichnet, dass** jedes der beiden Magnetelemente (56, 58; 60, 62; 64, 66) mindestens einen permanentmagnetische Körper umfasst.

4. Vorrichtung nach Anspruch 1 oder 2, **dadurch gekennzeichnet, dass** eines der beiden Magnetelemente mindestens einen ferromagnetischen Körper und das andere der beiden Magnetelemente mindestens einen permanentmagnetischen Körper umfasst.

5. Vorrichtung nach einem der vorangehenden Ansprüche, **dadurch gekennzeichnet, dass** mindestens eines der beiden Magnetelemente (56, 58; 60, 62; 64, 66) zwischen zwei Endstellungen beweglich ist.

6. Vorrichtung nach Anspruch 5, **dadurch gekennzeichnet, dass** das mindestens eine bewegliche Magnetelement (56, 58; 60; 66) entgegen der Federkraft einer Rückstellfeder (74, 82; 86; 96) beweglich ist.

7. Vorrichtung nach einem der vorangehenden Ansprüche, **dadurch gekennzeichnet, dass** der Betätigungsmechanismus (20) ein Schloss (36) mit einem zwischen der Schließstellung und der Offenstellung um eine Drehachse drehbaren Schlossteil (38) umfasst und dass mindestens eines der beiden Magnetelemente (56, 58; 60; 66) parallel zur Drehachse des Schlossteils (36) beweglich ist.

8. Vorrichtung nach Anspruch 7, **dadurch gekennzeichnet, dass** das bewegliche Magnetelement (58; 66) in der Blockierstellung des Schlosses (36) eine Drehung des Schlossteils (38) blockiert und in der Freigabestellung eine Drehung des Schlossteils (38) zulässt.

9. Vorrichtung nach einem der vorangehenden Ansprüche, **dadurch gekennzeichnet, dass** der Betätigungsmechanismus (20) ein Schloss (36) mit einem zwischen der Schließstellung und der Offenstellung um eine Drehachse drehbaren Schlossteil (38) umfasst und dass eines der beiden Magnetelemente (62) zusammen mit dem Schlossteil (38) um die Drehachse drehbar ist.

10. Vorrichtung nach einem der vorangehenden Ansprüche, **dadurch gekennzeichnet, dass** die beiden Magnetelemente (56, 58; 60, 62; 64, 66) jeweils ein im Portflansch (16) angeordnetes erstes Magnetelement (56; 60; 64) umfassen.

11. Vorrichtung nach Anspruch 10, **dadurch gekennzeichnet, dass** das erste Magnetelement (56; 60) im Portflansch (16) beweglich ist.

12. Vorrichtung nach Anspruch 10 oder 11, **dadurch gekennzeichnet, dass** die beiden Magnetelemente (56, 58) der zweiten Sicherheitseinrichtung (50) ein zweites Magnetelement (58) umfassen, das den Betätigungsmechanismus (20) in der Schließstellung freigibt, wenn beim Verbinden des Behälterflanschs (22) mit dem Portflansch (16) das erste Magnetelement (56) an das zweite Magnetelement (58) angenähert wird.

13. Vorrichtung nach Anspruch 10 oder 11, **dadurch gekennzeichnet, dass** die beiden Magnetelemente (60; 62) der dritten Sicherheitseinrichtung (52) ein zweites Magnetelement (62) umfassen, das sich beim Bewegen des Betätigungsmechanismus (20) in die Offenstellung vom ersten Magnetelement (60) weg bewegt, woraufhin das erste Magnetelement (60) den mit dem Portflansch (16) verbundenen Behälterflansch (22) blockiert.

14. Vorrichtung nach Anspruch 10 oder 11, **dadurch gekennzeichnet, dass** die beiden Magnetelemente (64; 66) der vierten Sicherheitseinrichtung (54) ein zusammen mit der Porttür (18) bewegliches zweites Magnetelement (66) umfassen, das den Betätigungsmechanismus (20) in der Offenstellung blockiert, wenn sich das zweite Magnetelement (66) beim Öffnen der Porttür (18) vom ersten Magnetelement (64) weg bewegt.

## Claims

1. Apparatus (10) for sterile transfer of material between a container and an isolator, wherein the container has a container opening surrounded by a container flange (22) and a container lid (24) removable from the container flange (22) for closing the container opening, wherein the isolator has a port opening surrounded by a port flange (16), a port door (18) for closing the port opening and an actuating mechanism (20) movable between a closed position and an open position for opening and closing the port door (18), wherein the container flange (22) is detachably connectable to the port flange (16) and the container lid (24) is detachably connectable to the port door (18), and to one or more of the following safety devices (50, 52, 54):
- a first safety device that blocks the actuating mechanism (20) in the closed position and releases it when the port door (18) rests against the container lid (24),
- a second safety device (50) that blocks the actuating mechanism (20) in the closed position and releases it when the container flange (22) is correctly connected to the port flange (16),
- a third safety device (52) that blocks the container flange (22) connected to the port flange (16) when the actuating mechanism (20) leaves the closed position, and that prevents the two flanges (16, 22) from being released from each other as long as the port door (18) is open,
- a fourth safety device that blocks return of the actuating mechanism (20) from the open position to the closed position when the port door (18) is open, and
- a fifth safety device, which blocks the actuating mechanism (20) if the container flange (22) is not correctly connected to the port flange (16) between the release of the actuating mechanism (20) by the second safety device (50) and the blocking of the container flange (22) by the third safety device (52), **characterised in that** at least one of the safety devices (50; 52; 54) comprises two magnetic fields (56, 58; 60, 62; 64, 66), which are movable relative to one another, **in that** at least one closed wall (68, 70) without a through-opening is arranged between the two magnetic elements (56, 58; 60, 62; 64, 66) and **in that** the magnetic elements (56, 58; 60, 62; 64, 66) attract or repel one another through the closed wall (68, 70) either in a blocking position or in a release position of the safety device (50; 52; 54).

2. Apparatus according to claim 1, **characterised in that** the two magnetic elements (56, 58; 60, 62; 64, 66) are arranged in two components (32; 16) of the apparatus, which components are separated by the at least one wall (68, 70) and can move in relation to one another.

3. Apparatus according to claim 1 or 2, **characterised in that** each of the two magnetic elements (56, 58; 60, 62; 64, 66) comprises at least one permanent magnetic body.

4. Apparatus according to claim 1 or 2, **characterised in that** one of the two magnetic elements comprises at least one ferromagnetic body and the other of the two magnetic elements comprises at least one permanent magnetic body.

5. Apparatus according to any one of the preceding claims, **characterised in that** at least one of the two magnetic elements (56, 58; 60, 62; 64, 66) is movable between two end positions.

6. Apparatus according to claim 5, **characterised in that** the at least one moveable magnetic element (56, 58; 60; 66) can be moved against the spring force of a return spring (74, 82; 86; 96).

7. Apparatus according to any one of the preceding claims, **characterised in that** the actuating mechanism (20) comprises a lock (36) with a lock part (38) rotatable about an axis of rotation between the closed position and the open position, and **in that** at least one of the two magnetic elements (56, 58; 60; 66) is movable parallel to the axis of rotation of the lock part (36).

8. Apparatus according to claim 7, **characterised in that** the movable magnetic element (58; 66) blocks rotation of the lock part (38) in the blocking position of the lock (36) and permits rotation of the lock part (38) in the release position.

9. Apparatus according to any one of the preceding claims, **characterised in that** the actuating mechanism (20) comprises a lock (36) with a lock part (38) rotatable about an axis of rotation between the closed position and the open position, and **in that** one of the two magnetic elements (62) is rotatable together with the lock part (38) about the axis of rotation.

10. Apparatus according to any one of the preceding claims, **characterised in that** the two magnetic elements (56, 58; 60, 62; 64, 66) each comprise a first magnetic element (56; 60; 64) arranged in the port flange (16).

11. Apparatus according to claim 10, **characterised in that** the first magnetic element (56; 60) is movable in the port flange (16).

12. Apparatus according to claim 10 or 11, **characterised in that** the two magnetic elements (56, 58) of the second safety device (50) comprise a second magnetic element (58), which releases the actuating mechanism (20) in the closed position when the first magnetic element (56) is brought closer to the second magnetic element (58) when the container flange (22) is connected to the port flange (16).

13. Apparatus according to claim 10 or 11, **characterised in that** the two magnetic elements (60; 62) of the third safety device (52) comprise a second magnetic element (62), which moves away from the first magnetic element (60) when the actuating mechanism (20) is moved to the open position, whereupon the first magnetic element (60) blocks the container flange (22) connected to the port flange (16).

14. Apparatus according to claim 10 or 11, **characterised in that** the two magnetic elements (64; 66) of the fourth safety device (54) comprise a second magnetic element (66), which is movable together with the port door (18) and which blocks the actuating mechanism (20) in the open position when the second magnetic element (66) moves away from the first magnetic element (64) when the port door (18) is opened.

## Revendications

1. Dispositif (10) pour le transfert stérile de produits entre un récipient et un isolateur, le récipient présentant une ouverture de récipient entourée par une bride de récipient (22) et un couvercle de récipient (24) détachable de la bride de récipient (22) pour fermer l'ouverture de récipient, l'isolateur présentant une ouverture de port entourée par une bride de port (16), une porte de port (18) pour fermer l'ouverture de port et un mécanisme d'actionnement (20) mobile entre une position de fermeture et une position d'ouverture pour ouvrir et fermer la porte de port (18), la bride de récipient (22) pouvant être reliée de manière amovible à la bride de port (16) et le couvercle de récipient (24) pouvant être relié de manière amovible à la porte de port (18), ainsi qu'à un ou plusieurs des appareils de sécurité suivants (50, 52, 54) :
- un premier appareil de sécurité qui bloque le mécanisme d'actionnement (20) dans la position de fermeture et le libère lorsque la porte de port (18) est en appui contre le couvercle de récipient (24),
- un deuxième appareil de sécurité (50) qui bloque le mécanisme d'actionnement (20) dans la position de fermeture et le libère lorsque la bride de récipient (22) est correctement reliée à la bride de port (16),
- un troisième appareil de sécurité (52) qui bloque la bride de récipient (22) reliée à la bride de port (16) lorsque le mécanisme d'actionnement (20) quitte la position de fermeture, et qui empêche le détachement des deux brides (16, 22) l'une de l'autre tant que la porte de port (18) est ouverte,
- un quatrième appareil de sécurité (54) qui bloque un retour du mécanisme d'actionnement (20) de la position d'ouverture à la position de fermeture lorsque la porte de port (18) est ouverte, et
- un cinquième appareil de sécurité qui bloque le mécanisme d'actionnement (20) lorsque la bride de récipient (22) n'est pas correctement reliée à la bride de port (16) entre la libération du mécanisme d'actionnement (20) par le deuxième appareil de sécurité (50) et le blocage de la bride de récipient (22) par le troisième appareil de sécurité (52), **caractérisé en ce qu'**au moins l'un des appareils de sécurité (50 ; 52 ; 54) comprend deux éléments magnétiques (56, 58 ; 60, 62 ; 64, 66) qui sont mobiles l'un par rapport à l'autre, **en ce qu'**entre les deux éléments magnétiques (56, 58 ; 60, 62 ; 64, 66) est agencée au moins une paroi fermée (68, 70) sans ouverture de passage et **en ce que** les éléments magnétiques (56, 58 ; 60, 62 ; 64, 66) s'attirent ou se repoussent mutuellement soit dans une position de blocage soit dans une position de libération de l'appareil de sécurité (50 ; 52 ; 54) à travers la paroi fermée (68, 70).

2. Dispositif selon la revendication 1, **caractérisé en ce que** les deux éléments magnétiques (56, 58 ; 60, 62 ; 64, 66) sont agencés dans deux composants (32 ; 16) du dispositif séparés par l'au moins une paroi (68, 70) et mobiles l'un par rapport à l'autre.

3. Dispositif selon la revendication 1 ou 2, **caractérisé en ce que** chacun des deux éléments magnétiques (56, 58 ; 60, 62 ; 64, 66) comprend au moins un corps magnétique permanent.

4. Dispositif selon la revendication 1 ou 2, **caractérisé en ce que** l'un des deux éléments magnétiques comprend au moins un corps ferromagnétique et l'autre des deux éléments magnétiques comprend au moins un corps magnétique permanent.

5. Dispositif selon l'une quelconque des revendications précédentes, **caractérisé en ce qu'**au moins l'un des deux éléments magnétiques (56, 58 ; 60, 62 ; 64, 66) est mobile entre deux positions d'extrémité.

6. Dispositif selon la revendication 5, **caractérisé en ce que** l'au moins un élément magnétique mobile (56, 58 ; 60 ; 66) est mobile à l'encontre de la force de ressort d'un ressort de rappel (74, 82 ; 86 ; 96).

7. Dispositif selon l'une quelconque des revendications précédentes, **caractérisé en ce que** le mécanisme d'actionnement (20) comprend une serrure (36) avec une partie de serrure (38) rotative autour d'un axe de rotation entre la position de fermeture et la position d'ouverture, et **en ce qu'**au moins l'un des deux éléments magnétiques (56, 58 ; 60 ; 66) est mobile parallèlement à l'axe de rotation de la partie de serrure (36).

8. Dispositif selon la revendication 7, **caractérisé en ce que** l'élément magnétique mobile (58 ; 66) bloque une rotation de la partie de serrure (38) dans la position de blocage de la serrure (36) et autorise une rotation de la partie de serrure (38) dans la position de libération.

9. Dispositif selon l'une quelconque des revendications précédentes, **caractérisé en ce que** le mécanisme d'actionnement (20) comprend une serrure (36) avec une partie de serrure (38) rotative autour d'un axe de rotation entre la position de fermeture et la position d'ouverture, et **en ce que** l'un des deux éléments magnétiques (62) est rotatif conjointement avec la partie de serrure (38) autour de l'axe de rotation.

10. Dispositif selon l'une quelconque des revendications précédentes, **caractérisé en ce que** les deux éléments magnétiques (56, 58 ; 60, 62 ; 64, 66) comprennent chacun un premier élément magnétique (56 ; 60 ; 64) agencé dans la bride de port (16).

11. Dispositif selon la revendication 10, **caractérisé en ce que** le premier élément magnétique (56 ; 60) est mobile dans la bride de port (16).

12. Dispositif selon la revendication 10 ou 11, **caractérisé en ce que** les deux éléments magnétiques (56, 58) du deuxième appareil de sécurité (50) comprennent un deuxième élément magnétique (58) qui libère le mécanisme d'actionnement (20) dans la position de fermeture lorsque, lors de la liaison de la bride de récipient (22) à la bride de port (16), le premier élément magnétique (56) est rapproché du deuxième élément magnétique (58).

13. Dispositif selon la revendication 10 ou 11, **caractérisé en ce que** les deux éléments magnétiques (60 ; 62) du troisième appareil de sécurité (52) comprennent un deuxième élément magnétique (62) qui s'éloigne du premier élément magnétique (60) lors du déplacement du mécanisme d'actionnement (20) vers la position d'ouverture, après quoi le premier élément magnétique (60) bloque la bride de récipient (22) reliée à la bride de port (16).

14. Dispositif selon la revendication 10 ou 11, **caractérisé en ce que** les deux éléments magnétiques (64 ; 66) du quatrième appareil de sécurité (54) comprennent un deuxième élément magnétique (66) mobile conjointement avec la porte de port (18), qui bloque le mécanisme d'actionnement (20) dans la position d'ouverture lorsque le deuxième élément magnétique (66) s'éloigne du premier élément magnétique (64) lors de l'ouverture de la porte de port (18).
